# EUROPEAN PATENT APPLICATION

(11) **EP 1 502 554 A2**
(43) Date of publication of application: **02.02.2005**
(21) Application number: 04076559.6
(22) Date of filing: 27.05.2004
(51) Int. Cl.: A61B 18/02

(54) **Cryoablation system**

(30) Priority: 31.07.2003 US 631485
(71) Applicant: Cryocor, Inc., San Diego, California 92121 (US)
(72) Inventor: Castellano, Thomas, Temecula, California 92591 (US); Lentz, David J., La Jolla, California 92037 (US); Ryba, Eric, San Diego, California 92122 (US)
(74) Representative: Shortt, Peter Bernard

(57) **Abstract**

Systems and methods for treating an arrhythmia originating in a pulmonary vein of a patient are described. The system includes a rigid sheath (400,500) and an elongated catheter (102,402,502) that defines an axis and has an ablating distal section. The distal section includes a plurality of conductive bands (108,412,506), with each band establishing an enclosed chamber (118). The ablating distal section is reconfigurable between a first compact configuration in which each band is positioned relatively near the axis for transit through the sheath and second expanded configuration in which each band is positioned relatively far from the axis. Once in the second configuration, a fluid refrigerant is expanded into each enclosed chamber to cool the bands and cryoablate tissue. The second configuration is particularly useful for ablating a circumferential band of tissue, for example, a band of tissue surrounding the opening (i.e. ostium) where a pulmonary vein connects with the left atrium.

## Description

### FIELD OF THE INVENTION

The present invention relates to devices and methods for the treatment of cardiac arrhythmia, and more specifically relates to devices and methods for the treatment of focal atrial arrhythmia using cryoablation.

### BACKGROUND OF THE INVENTION

Normal cardiac rhythm is maintained by precisely timed nerve signals conducted through cardiac tissue to electrically stimulate synchronous contractions of the four heart chambers (2 ventricles and 2 atria). In a normal rhythm, the nerve signals are typically conducted along paths initiating at the sino-atrial (SA) node and passing from there through the atrioventricular (AV) node and the bundle of His to the ventricular myocardial tissue.

Abnormal cardiac rhythms, or arrhythmias, including atrial fibrillation, are potentially dangerous medical conditions which may result from disturbances in the site of origin and/or the pathways of conduction of the nerve impulses that excite contraction of the four chambers of the heart. The site of origin and pathways of conduction of these signals are currently mapped, for example using an electrocardiograph (ECG) in conjunction with mapping methods such as those described in U.S. Patent No. 4,641,649 to Walinsky et al.

One common type of abnormal atrial fibrillation occurs when the contraction initiating signals originate within one or more of the pulmonary veins, rather than at the SA node. These atrial arrhythmias have been treated by a variety of methods including pharmacologic treatments, highly invasive surgical procedures and linear and circumferential radio frequency (RF) ablations of the myocardial wall. However, each of these methods has drawbacks, e.g., the pain and extended recovery time for invasive surgery, relative ineffectiveness of pharmacologic treatments and restenosis at the ablation site due to the application of RF energy or other heat based therapies, and the necessity to repeat the ablation procedure to treat a sufficiently large area of tissue.

### SUMMARY OF THE INVENTION

The present invention is directed to systems and methods for ablating tissue of a patient. For the present invention, the system includes a substantially rigid sheath and an elongated cryoablation catheter that defines an axis. The catheter has an ablating distal section that includes a plurality of conductive bands, with each band establishing an enclosed chamber. For the cryoablation catheter, the ablating distal section is reconfigurable between a first configuration and a second configuration. In the first configuration, each band is positioned relatively near the axis to place the ablating distal section in a relatively compact configuration for transit through the sheath. On the other hand, when the ablating distal section is in the second configuration, each band is positioned relatively far from the axis. The second configuration is useful for ablating a circumferential band of tissue, for example, a band of tissue surrounding the opening (i.e. ostium) where a pulmonary vein connects with the left atrium.

Once the ablating distal section is in the second configuration, the bands can be placed in contact with selected tissue and cooled to cryoablate the tissue. To cool the bands, a fluid refrigerant is expanded into each enclosed chamber. In greater detail, the fluid refrigerant can be passed through one or more orifices to expand the fluid and cool the bands.

In a first embodiment of the system, the ablating distal section includes a plurality of cylindrical bands that are each centered on the catheter axis. A capillary tube that is positioned along the axis is formed with one or more orifices and establishes one or more chambers between the inner surface of each band and the outer surface of the capillary tube. Refrigerant is pumped through the capillary tube for outflow into the chamber(s) through the orifice(s). In one implementation, the plurality orifices are arranged along a line that is parallel to the axis, with a proximal orifice having a relatively large diameter and a distal orifice having a relatively small diameter. The remaining orifices (i.e. the orifices between the distal and proximal orifices) have diameters that progressively decrease, from orifice to orifice, in a distal direction. This cooperation of structure is provided to maintain a constant cooling rate along the ablating distal section.

In the first, compact configuration that is useful for passing the ablative distal section through the sheath, the bands combine to form a cylinder and the capillary tube is substantially straight. On the other hand, in the second configuration, the bands combine to form a coiled structure and the capillary tube typically bends and becomes arcuate. To reconfigure the ablating distal section, a shape memory element which has an arcuate shape when unconstrained can be attached to the bands. With this cooperation of structure, the shape memory element can be deformed (e.g. elastically deformed) until it is straight or only slightly curved, placing the ablating distal section in the first, compact configuration. While the shape memory element is deformed, the ablating distal section can be inserted into and advanced through the sheath, where the sheath acts to constrain the shape memory element. When the ablating distal section exits the distal end of the sheath, the shape memory element becomes unconstrained and assumes its arcuate shape, reconfiguring the ablating distal section into the second, substantially coiled configuration. The coiled ablating distal section can then be used to cryoablate a circumferentially shaped band of tissue in a one-step process. Alternatively, a linear actuator (e.g. pull wire) having a distal end attached to the ablating distal section can be manipulated at an extracorporeal location to reconfigure the ablating distal section into a coiled configuration.

In another embodiment of the system, the ablating distal section includes a plurality of arms with each arm extending from a proximal end to a distal end and having a hinge joint therebetween. For this embodiment, a band is mounted on each arm between the arm's hinge joint and arm's distal end. A linear actuator (e.g. pull rod) is attached to the distal end of each arm to proximally retract the distal end of each arm relative to the arm's proximal end. In the first configuration, each arm is somewhat straight and the bands are typically positioned very close to the catheter axis. When the rod is pulled, each arm bends at its respective hinge joint causing each band to move radially outward from the axis. This reconfigures the ablating distal section into the second configuration suitable for cryoablating tissue.

In another embodiment, the ablating distal section includes a plurality of arms, with a band attached to the distal end of each arm. Each arm, when unconstrained, has an arcuate shape. Specifically, when unconstrained, the proximal end of each arm is positioned near the axis and the distal end of each arm deflects from the catheter axis to distance each band from the axis. With this cooperation of structure, the arms can be deformed (e.g. elastically deformed) until they are straight or only slightly curved, placing the ablating distal section in the first compact configuration. With the arms deformed, the ablating distal section can be inserted into and advanced through the sheath, with the sheath constraining the arms. When the ablating distal section is pushed out of the distal end of the sheath, the arms become unconstrained and assume their arcuate shape, reconfiguring the ablating distal section into the second, expanded configuration. With the ablating distal section expanded, the bands can be placed in contact with target tissue and cooled to cryoablate the contacted tissue.

In one application of the present invention, the distal end of the sheath is inserted into a patient's vascular system and advanced into the right atrium. The interatrial septum is then pierced and the distal end of the sheath is passed through the septum and into the left atrium. In one implementation, the distal end of the rigid sheath is then maneuvered into a position proximal to a portion of the tissue to be ablated. Next, the distal end of a cryoablation catheter having an ablating distal section, such as one of the ablating distal sections described above, is pushed through the sheath until the ablating distal section is pushed out of the distal end of the sheath. For some embodiments, the ablating distal section reconfigures into an expanded, and in some cases coiled, second configuration as the ablating distal section exits the sheath (see discussion above). For other embodiments, a linear actuator (e.g. a pull wire or pull rod) can be activated once the ablating distal section exits the sheath to reconfigure the ablating distal section into an expanded, and in some cases coiled, second configuration.

Once the ablating distal section has been placed in the second configuration, the bands are placed in contact with the tissue to be ablated and cooled. Specifically, a refrigerant is passed through the orifices in the ablating distal section to expand the refrigerant into the chamber(s) to cool the bands and contacted tissue. For example, the contacted tissue can be a circumferential band of tissue surrounding the opening (i.e. ostium) where one of the pulmonary veins connects with the left atrium.

In another implementation, the sheath is formed with a curved distal portion. The curved distal portion is advanced into the left atrium after piercing the interatrial septum. Once in the left atrium, the distal portion of the sheath is maneuvered until the distal end is facing a selected pulmonary vein opening. Next, the ablating distal section of a cryoablation catheter is passed through the sheath and into the selected opening where the pulmonary vein connects with the left atrium. The bands are then placed in contact with the tissue to be ablated and cooled.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figures 1 and 1a are side elevation views showing an embodiment of a cryoablation catheter according to the present invention;
Figures 2 and 2a are cross sectional views showing details of the embodiment shown in Figure 1;
Figure 3 is a cross sectional view on line III-III of the embodiment shown in Figure 1;
Figure 4 is a side view showing a second embodiment of the cryoablation catheter according to the present invention;
Figure 5 is a side view showing a third embodiment of the cryoablation catheter according to the present invention;
Figure 6 is a side view showing a fourth embodiment of the cryoablation catheter according to the present invention;
Figure 7 is a side view showing an embodiment of the device according to the present invention in position for penetrating the foramen ovale of a patient;
Figure 8 is a side view showing the device of Figure 7 in position with a dilator penetrating the opening in the foramen ovale;
Figure 9 is a side view showing the device of Figure 7 in position within a pulmonary vein; and
Figure 10 is a side view showing the device of Figure 7 in position within the pulmonary vein, with the catheter deployed.

### DESCRIPTION OF THE PREFERRED EMBODIMENTS

The present invention may be further understood with reference to the following description and the appended drawings, wherein like elements are referred to with the same reference numerals.

In many cases, arrhythmia results from contraction initiating signals that originate within one or more of the pulmonary veins rather than from the SA node. Known techniques may be used to locate the point of origin of the aberrant signals, and their paths of conduction. Once these locations have been determined, the device and method according to the present invention may be employed to ablate a portion of tissue within the identified pulmonary vein between the source of the signals and the left atrium, e.g., near the opening or collar of the pulmonary vein, to create a circumferential conduction block within the pulmonary vein. This conduction block prevents the abnormal contraction originating signals from propagating into the left atrium to restore a normal contraction sequence.

The present method and apparatus allows for ablation of an elongated strip of tissue (e.g. a linear ablation) and can reduce the number of applications required to create the circumferential conduction block, thereby reducing the time required to complete the procedure as well as the trauma to the patient.

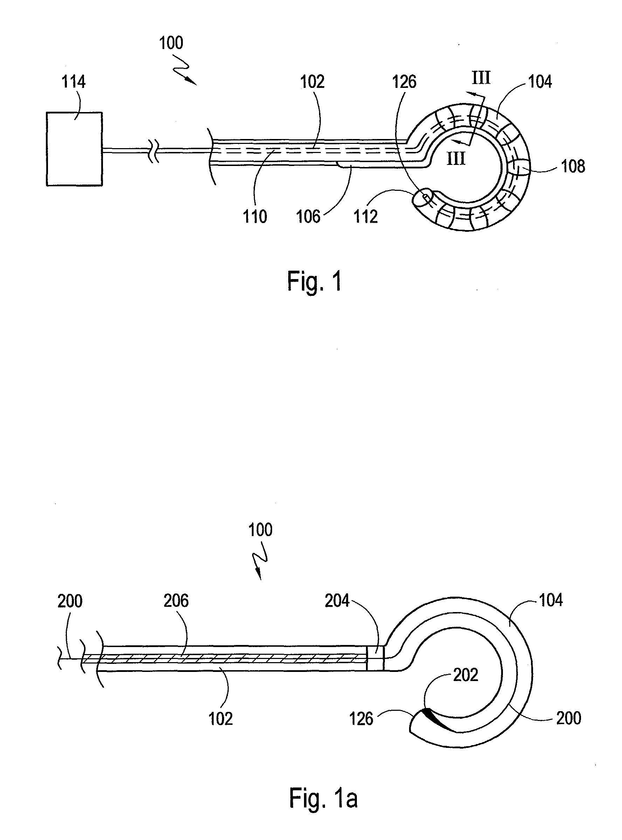

Figure 1 shows a portion of the catheter according to the present invention that is placed in contact with a portion of tissue to be treated. The cryoablation device 100 in this exemplary embodiment is designed to extend from a sheath, not shown in Figure 1, after the sheath has pierced the septum of the heart, and has been positioned near the tissue to be treated. Cryoablation device 100 includes a catheter 102 that may be extended and manipulated as more fully described in the '657 patent.

Catheter 102 may include a spiral portion 104 which may be shaped to match a surface of the portion of tissue to be ablated. In one exemplary embodiment, the spiral portion 104 is maintained in a straight configuration while received within the sheath, and deploys to the spiral configuration only after being pushed outside the sheath. A shape memory element 106 may urge the spiral portion 104 to assume the desired configuration once the spiral portion 104 has left the sheath and is no longer restrained thereby. Shape memory element 106 may, for example, be a stylet made of Nitinol, which can be both external or internal to the catheter 102. In an exemplary use, the catheter 102 may be straightened by a user outside of the patient, before being introduced into the sheath, and remains straight while therewithin. Once the catheter 102 has been pushed out of the distal end of the sheath, the shape memory element 106 returns to its unconstrained shape, and causes the spiral portion 104 to assume the desired shape.

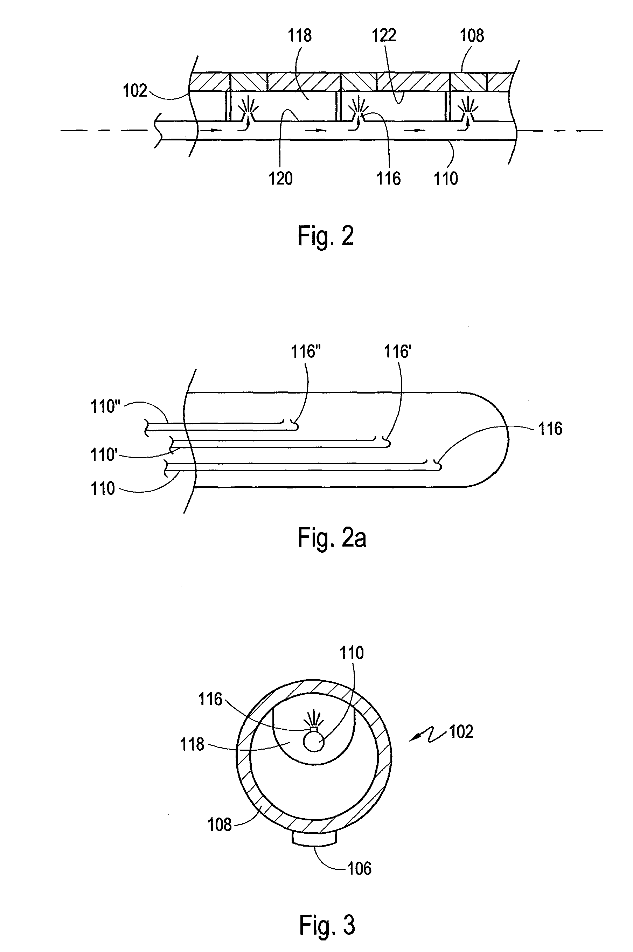

The exemplary embodiment of the catheter 102 according to the present invention shown in Figures 1 and 2 may also include a pull wire coupled to a distal end thereof and extending through the catheter 102 to a proximal end thereof so that, when a user pulls the pull wire proximally, the distal tip of the catheter 102 is pulled toward the shaft, to control the shape or size of the spiral portion 104. As shown in Figure 1 a, pull wire 200 extends from a proximal end of the catheter 102 to the distal tip 126 of the spiral portion 104. In one exemplary embodiment, pull wire 200 is attached to spiral portion 104 at a point 202 by welding or by another secure method. Pull wire 200 may be pulled proximally or released by an operator to control the size or diameter of spiral section 104, to fit the orifice of a particular pulmonary vein. A tip union 204 may be included to provide a support for pull wire 200, so that all the tension is applied to the spiral section 104. Pull wire 200 may be contained within a tubular sheath 206, which can resist compression along the length of pull wire 200, but which allows bending of catheter 102. The purpose of sheath 206 is to prevent bowing or snaking of catheter 102 when tension is applied to pull wire 200. In one exemplary embodiment, sheath 206 may be formed by a coil spring.

Cross referencing Figure 1 and 2 shows that one or more thermally conductive bands 108 may also be included on the surface of catheter 102. According to the exemplary embodiment of the invention, the bands 108 may preferably be made of a material that efficiently conducts heat, for example gold plated copper. As shown more clearly in Figure 2, the catheter 102 may include a capillary tube or lumen 110 that extends from near the distal portion 112 of catheter 102 to a portion of the catheter 102 which remains outside the patient's body during use. The capillary tube 110 may be connected to a source of refrigerant fluid 114 that provides a flow of refrigerant fluid to the catheter 102. The refrigerant fluid may be a hydrocarbon refrigerant, nitrous oxide, or a mixed gas refrigerant.

The capillary tube 110, in the exemplary embodiment shown in Figure 2, includes a plurality of orifices 116, each opening into a corresponding expansion chamber 118. Expansion chambers 118 may be defined by an outer surface 120 of the capillary tube 110 and an inner surface 122 of the bands 108. Those skilled in the art will understand that all of the orifices 116 may open into a single large expansion chamber 118 extending along the length of the spiral portion 104. As will be understood by those of skill in the art, refrigerant fluid provided at high pressure within the capillary tube 110 expands through the orifices 116 creating a Joule Thompson cooling effect and lowering the temperature within the chamber 118. Since the bands 108 are made of thermally conductive material while the rest of the surface of the catheter 102 is formed of thermally insulating material, the cooling from the Joule Thompson effect is substantially directed to the portion of the surface of the catheter 102 (i.e., the bands 108) that is in contact with the portion of tissue to be ablated.

Figure 3 shows a cross sectional view of catheter 102, taken along line III-III of Figure 1. An exemplary embodiment of an expansion chamber 118 is depicted, with orifices 116 opening through the capillary tube 110 to allow expansion of the refrigerant fluid therefrom. As the refrigerant fluid expands out of the orifices 116 into the chamber 118, it cools down and in turn cools down bands 108. Bands 108 are disposed along catheter 102 so that the tissue ablated by adjacent bands 108 overlaps to form a continuous strip of ablated tissue along the length of the spiral portion 104. As shown in Figure 2, in order to maintain a degree of cooling substantially equal along the length of the spiral portion 104, the opening area of the orifices 116 decreases from a maximum size for the proximal-most orifice 116 to a minimum size of the distal-most orifice 116. Thus, as would be understood by those of skill in the art, although the pressure in the capillary tube 110 decreases from the proximal-most orifice 116 to the distal-most orifice 116, as the size of the openings is correspondingly decreased, the velocity of the refrigerant gas exiting the orifices 116 remains substantially the same, and thus, the cooling effect remains substantially constant along the length of the spiral portion 104.

In a different exemplary embodiment according to the present invention, the cooling along the length of the ablating distal section may be balanced by providing orifices 116 having the same dimensions, each having an independent supply of refrigerant fluid. For example, as shown in Figure 2a, capillary tubes 110, 110' and 110" extend from a refrigerant source (not shown) to a single corresponding orifice 116, 116' and 116". Since each capillary tube 110, 110' and 110" only supplies refrigerant to one orifice 116, 116' and 116", the pressure of the refrigerant reaching each of the orifices, 116 116' and 116" is substantially the same, and the respective bands 108 undergo substantially the same amount of cooling.

A conductive distal tip 126 may also be included in the catheter 102, located at the most distal portion of the ablating section, according to an embodiment of the invention. Tip 126 may be used to ablate pinpoint portions of tissue, or may combine with the bands 108 to cryoablate an extended band of tissue around the circumference of the pulmonary vein in a single application. As described above, the tip 126 is cooled by refrigerant fluid from the capillary tube 110 expanded through a distal-most orifice 116.

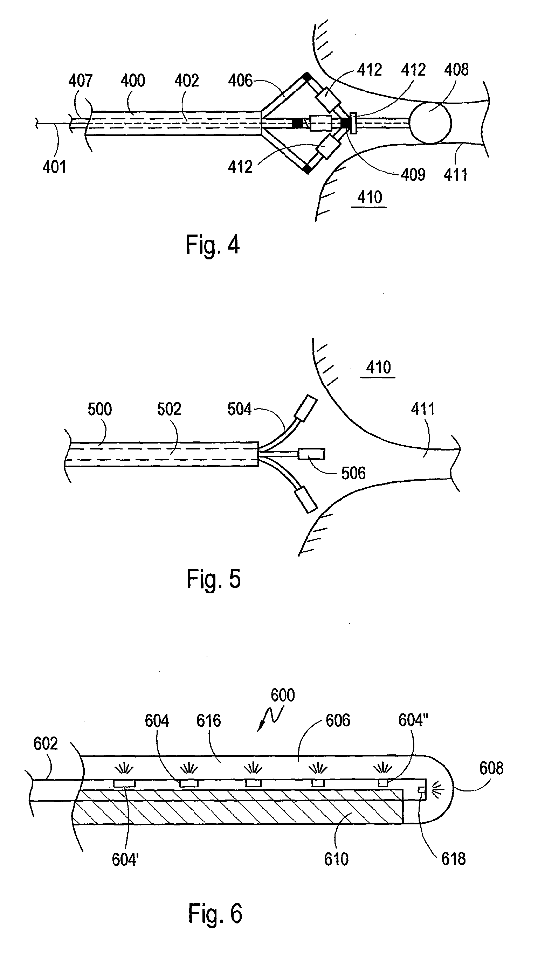

Figure 4 shows another exemplary embodiment of a cryoablation catheter according to the present invention. Catheter 402 is shown in a deployed configuration extending from a sheath 400, in position adjacent to an opening 410 of a pulmonary vein 411. The catheter 402 includes extensible arms 406 that deploy in an operative, basket-like, configuration as shown in Figure 4 when pushed out of sheath 400. Arms 406 include conductive bands 412 that are cooled by expansion of a refrigerant fluid, as described above in regard to Figs. 1 and 2. In the exemplary embodiment of Figure 4, the distal ends of arms 406 are coupled to one another at a common joint 409. A distal end of rod 407 is also connected to arms 406 at joint 409, such that rod 407 can slide longitudinally with respect to sheath 400. Thus, after the arms 406 have been moved distally out of the sheath 400, an operator may deploy the arms 406 to the operative position by pulling the rod 407 proximally, to draw proximally the joint 409 where arms 406 are coupled to one another. This causes the arms 406 to bow outward radially from the axis of the catheter 402 so that the conductive bands 412 are spaced circumferentially and face distally toward the tissue to be ablated. In the operative position of arms 406, conductive bands 412 are deployed in a two-dimensional or a three-dimensional array. For example, there can be four arms 406 in an orthogonal configuration, each with a conductive band 412. A fifth conductive band 412 may be located centrally, at joint 409.

Once the arms 406 are deployed, the user advances the catheter 402 distally until the conductive bands 412 are in contact with the portion of tissue to be ablated. Cooling is initiated and maintained until the tissue adjacent to each of the conductive bands 412 is ablated. The user then allows the conductive bands 412 to return to body temperature so that they may be removed from contact with the tissue without harm thereto. If further ablation is required to complete a circumferential conduction block, the user rotates the catheter 402 around the axis thereof so that the conductive bands 412 are offset from their former positions, and the process is repeated until the circumferential conduction block is complete. Then, when the operation has been completed, the arms 406 may be returned to the collapsed configuration by simply withdrawing the catheter 402 into the sheath 400 or by extending rod 407 distally.

As would be understood by those of skill in the art, the arms 406 may alternatively be deployed to the operative position by a stylet as described above, or by a different mechanism that may include shape memory and/or resilient elements as would be understood by those of skill in the art. As shown in Figure 4, the device may, for example, include four arms 406 disposed equiangularly around the axis of the catheter 402. However, those skilled in the art will understand that different configurations with more or fewer arms may be used.

The exemplary embodiment shown in Figure 4 includes an occluding structure 408 that may be used to occlude the flow of blood through pulmonary vein 411. In one embodiment, the occluding structure 408 may be a balloon inflated by providing an inflation fluid through an inflation lumen 401 of the catheter 402. Once the catheter 402 has been properly positioned adjacent to the tissue to be treated, the occluding structure 408 is inflated to reduce or stop the flow of blood near the conductive bands 412. This reduces the heat transfer between the blood, the inner surfaces of the pulmonary vein 411 and the conductive bands 412. The expanding refrigerant fluid flowing in the capillary tube 110 is thus required to remove less heat to cool the tissue to a desired temperature, making the ablation process more efficient.

Figure 5 shows another embodiment of the cryoablation catheter according to the present invention. The catheter 502 is shown in the deployed configuration, after exiting sheath 500. Deployable arms 504 are shown in the operative configuration, such that conductive bands 506 are spaced from the axis of the catheter 502, azimuthally spaced from one another and facing a circumferential region of tissue to be ablated within the pulmonary vein 411. As in the embodiments described above, in an initial configuration, the arms 504 are constrained within the sheath 500, which is positioned near the tissue to be ablated. Once the desired position has been reached, the catheter 502 is pushed distally out of the sheath 500 and the arms 504 deploy under the force of, for example, memory shape elements such as those described above. Refrigerant fluid is then used to cool conductive bands 506, as described above, to create a circumferential conduction block.

An ablation element 600 according to a further embodiment of the invention shown in Figure 6 may be used as a stand alone medical device, for example, during open heart surgery to ablate selected portions of tissue to treat cardiac arrhythmias. Ablation element 600 may preferably be plastically deformable in this embodiment so that a user may bend the ablation element 600 into a desired shape which desired shape would be retained during use of the ablation element 600. For example, the ablation element 600 may be formed of copper tubing so that it may be bent into a desired shape which shape will be retained during use. The ablation element 600 includes a capillary tube 602 that provides refrigerant fluid to the distal end of the ablation element 600 as described above in regard to the catheter embodiments. The capillary tube 602 includes a plurality of orifices 604 that permit the refrigerant fluid to expand out of the capillary tube 602 to cool the ablation element 600 to a desired temperature. The orifices 604 are disposed along the length of a portion of the capillary tube 602 facing a side of the ablation element 600 to be placed in contact with the tissue to be treated. As described above, the orifices 604 may be of different dimensions, for example, decreasing in size from a proximate portion of the ablation element 600 to a distal portion thereof, to compensate for decreasing pressure of the refrigerant fluid along the capillary tube 602. As shown in Figure 6, the orifice 604' is the furthest from tip 608, and has a larger diameter than orifice 604", that is nearest to tip 608.

As described above, an outer surface 606 of the ablation element 600 may be formed of, for example, copper or another thermally conductive material. The outer surface 606 is cooled by the refrigerant fluid expanding within the chamber 616 to provide a substantially uniformly cooled cryoablation surface for ablating tissue. In one exemplary embodiment, an additional orifice 618 may be provided at a distal end of the capillary tube 602 to cool a distal tip 608 of the ablation element 600. This allows a user to ablate specific points of tissue by applying the distal tip 608 thereto. Furthermore, the outer surface 606 may have a thermally insulating coating applied to predetermined portions thereof so that the cooling effect is substantially directed toward that portion of the outer surface 606 which is to contact the tissue to be ablated. For example, a coating of Pebax™ may be applied by RF fusion techniques to surfaces facing away from the orifices 604. Alternatively, an insulating cover may be provided to surround selected portions of the ablation element 600 while leaving the tissue contacting portions of the outer surface 606 exposed. That is, as with the insulative coating described above, the insulating cover may cover parts of the ablation element 600 that are not intended to contact the tissue to be ablated. Thus, the insulating cover 610 concentrates the cooling effect of the expanding refrigerant fluid at the tissue contacting portions of the ablation element 600 thereby reducing the amount of cooling required. Furthermore, those of skill in the art will understand that an insulative coating or cover as described herein may also be employed in any of the previously described catheter-based embodiments.

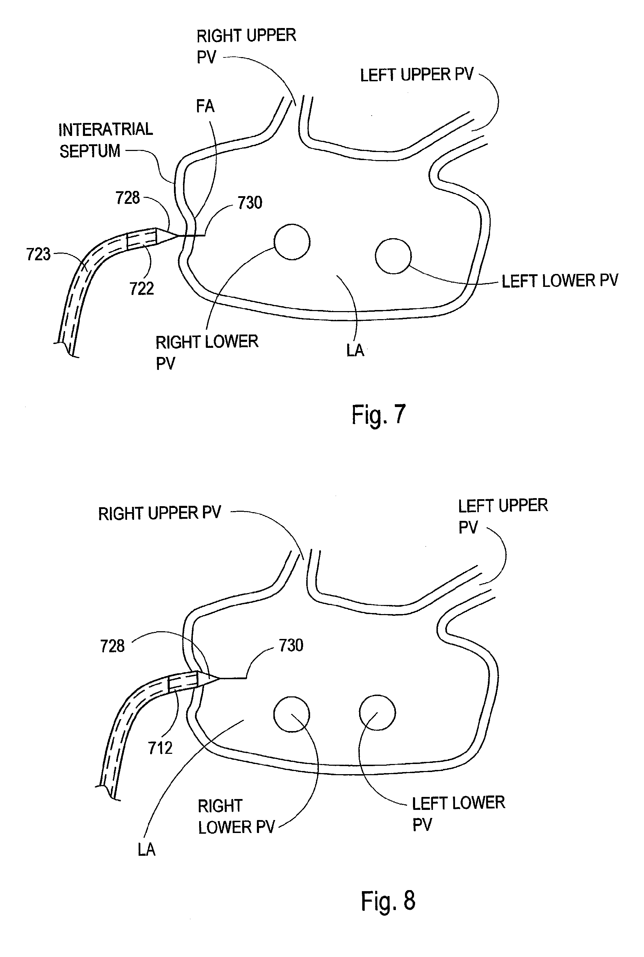

Figures 7-10 show a sequence of steps that may be used to introduce any of the cryoablation catheters described in regard to Figs. 1-5, into the heart of a patient. Those skilled in the art will understand that, although the catheter illustrated more closely resembles the catheter 100 of Figure 1, the same steps may be applied to use of any of the catheters of Figs. 1-5. As shown in Figure 7, a dilator 728 at the end of sheath 722 may include a Brockenbrough needle 730. The assembly is inserted through a central lumen 723 of the rigid sheath 722 until a distal end of the dilator 728 extends beyond a distal end of the rigid sheath 722. The user may then probe the interatrial septum noting the relative strength of various locations on the interatrial septum, until the precise location of the foramen ovale (FO) is determined (i.e., the FO forms a soft apical spot on the septum). Those skilled in the art will understand that intracardiac ultrasound may also be used to assist in locating the FO. Then the Brockenbrough needle 730 is extended from the distal end of the dilator 728 to pierce the FO forming a transeptal puncture (TP) extending into the left atrium (LA) as shown in Figure 7. The dilator 728 is then advanced through the TP in the interatrial septum to expand a diameter of the TP as shown in Figure 8.

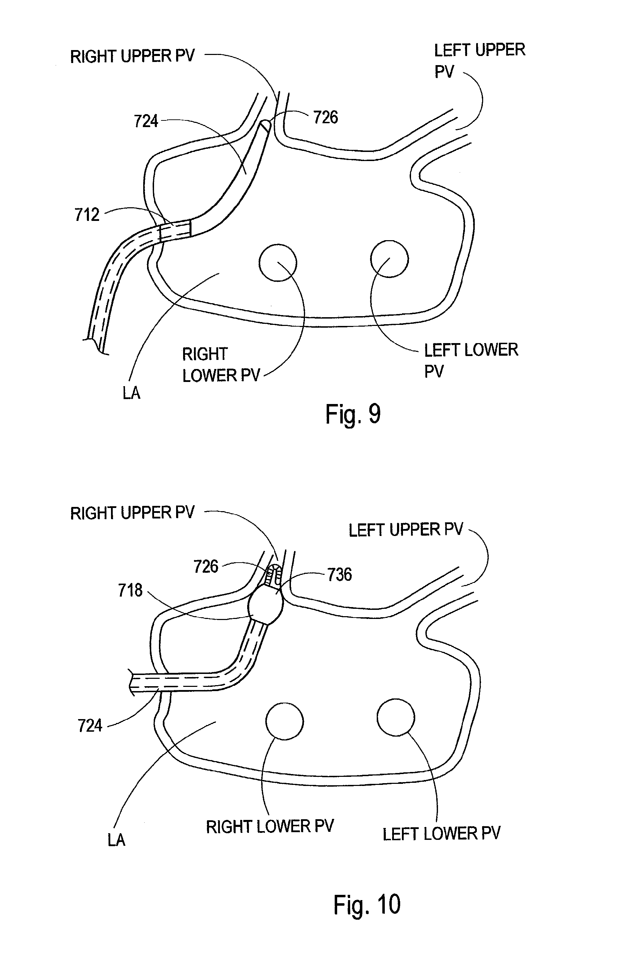

Thereafter, the Brockenbrough needle 730 is retracted into the dilator 728 and removed from the body. The rigid sheath 722 is then advanced along the dilator 728 to pass through the TP into the LA. A flexible section 712, which may for example be constructed in accord with the teaching of the '117 application, is then pushed along the rigid sheath 722 (utilizing the longitudinal rigidity of the flexible section 712) until a distal end of the flexible section 712 extends through the opening in the interatrial septum and into the LA, as shown in Figure 9. The dilator 728 may then be removed from the patient.

The ablation catheter 724 is then advanced distally through the rigid sheath 722 until the cryogenic tip 726 extends distally beyond the distal end of the rigid sheath 722 and the distal end of the flexible section 712. Several known techniques may be used for maneuvering catheter 724 to a desired position within the opening of the one of the PV's from which the contraction origination signals are improperly originating. In certain embodiments, the catheter 724 is deflectable via a deflection mechanism associated with the catheter handle, which may ease the positioning of the catheter. Furthermore, by advancing the rigid sheath 722 further into the LA, a pre-formed bend in the tip of the rigid sheath 722 may be employed to assist in aiming the cryogenic tip 726 toward the desired PV opening. After the cryogenic tip 726 has been properly positioned well within the PV, the flexible section 712 is advanced distally along the ablation catheter 724 until the distal end of the flexible section 712 is near the orifice at which the PV opens into the LA. To aid in ensuring proper positioning of the cryogenic tip 726 and the flexible section 712 in the orifice of the PV, the flexible section 712 and the rigid sheath 722 may include radiopaque markers at the respective distal ends thereof, or at other desired locations.

Once the flexible section 712 has been positioned near the opening of the PV, the user may inject contrast media into the PV via the flexible section 712. The contrast media may exit the flexible section 712 via openings 736 of the flexible section 712. This may be done to aid in locating, under fluoroscopic imaging, the orifice of the PV. The user may then inflate the balloon 718 to occlude the PV. In one embodiment, the inflation fluid may be a diluted contrast media solution such that the balloon 718 may more easily be seen under imaging. The flexible section 712 is then advanced until the balloon 718 is seated on the orifice of the PV, thereby occluding the flow of blood from the PV into the LA as shown in Figure 10. The description herein of a balloon 718 does not imply that blood flow must be occluded by an inflatable cuff. Rather, any structure which is radially extendible to occlude blood flow will serve the purposes of this invention. There are many alternative constructions for this structure, which will be known to those skilled in the art.

In the preceding specification, the present invention has been described with reference to specific exemplary embodiments thereof. It will, however, be evident that various modifications and changes may be made thereto without departing from the broadest spirit and scope of the present invention as set forth in the claims that follow. The specification and drawings are accordingly to be regarded in an illustrative rather than restrictive sense. For example, while the invention has been described for use with PV ablation, the device may be used in other parts of the vascular system.

While the particular cryoablation systems and methods as herein shown and disclosed in detail are fully capable of obtaining the objects and providing the advantages herein before stated, it is to be understood that they are merely illustrative of the presently preferred embodiments of the invention and that no limitations are intended to the details of construction or design herein shown other than as described in the appended claims.

## Claims

1. A system for ablating tissue of a patient, comprising:
a substantially rigid sheath;
an elongated cryoablation catheter defining an axis, the catheter having an ablating distal section including a plurality of conductive bands that form at least one partially enclosed chamber, the ablating distal section being reconfigurable between a first configuration for transit through the sheath wherein each band is positioned relatively near the axis and a second configuration for tissue ablation wherein each band is relatively far from the axis; and
a means for expanding a fluid refrigerant into each chamber to cool the bands and ablate the tissue.

2. The system as recited in claim 1 wherein the ablating distal section comprises a shape memory element having an arcuate shape when unconstrained.

3. The system as recited in claim 1 wherein the ablating distal section is coiled in the second configuration.

4. The system as recited in claim 1 wherein the catheter further comprises a linear actuator extending from the ablating distal section to a proximal end of the catheter to control reconfiguration of the ablating distal section.

5. The system as recited in claim 1 wherein the ablating distal section comprises a plurality of arms, each arm extending from a proximal end to a distal end and having a hinge joint therebetween with a band mounted on each arm between the hinge joint and distal end of each arm, and wherein the catheter further comprises a means for proximally retracting the distal end of each arm relative to the proximal end of the arm to reconfigure the ablating distal section into the second configuration.

6. The system as recited in claim 1 wherein the catheter further comprises an insulating cover disposed on selected portions of the ablating distal section.

7. The system as recited in claim 1 wherein the expanding means comprises a first orifice having diameter, D₁ and a second orifice positioned distal to the first orifice and having diameter, D₂, with D₁ > D₂.

8. The system as recited in claim 1 further comprising an occluding structure to occlude blood flow past the tissue to be ablated.

9. The system as recited in claim 8 wherein the occluding structure is an inflatable balloon.
